# EUROPEAN PATENT APPLICATION

(11) **EP 3 682 852 A1**
(43) Date of publication of application: **22.07.2020**
(21) Application number: 20152497.2
(22) Date of filing: 17.01.2020
(51) Int. Cl.: A61F 2/24

(54) **POSITION-ADJUSTABLE DEVICE FOR TREATMENT OF TRICUSPID REGURGITATION**

(30) Priority: 17.01.2019 KR 20190006359; 13.05.2019 KR 20190055911
(71) Applicant: Taupnumedical Co. Ltd., Busan 609-836 (KR)
(72) Inventor: KIM, June-hong, 48516 Busan (KR); PARK, Kyone Peter, 50653 Yangsan (KR); CHON, Min Ku, 50658 Yangsan-si (KR); CURLEY, Jimmy, 50658 Yangsan-si (KR)
(74) Representative: HGF

(57) **Abstract**

A position-adjustable device for treatment of tricuspid regurgitation is proposed. The position-adjustable device for the treatment of tricuspid regurgitation is used to verify whether heart failure of the right ventricle may occur, and includes: a first catheter provided with a first lumen; a second catheter inserted into the first lumen and provided with a second lumen into which a guidewire is inserted; an adjusting part coupled to a lower side of the first catheter and the second catheter, wherein the adjusting part is provided with a first direction-adjusting device adjusting a direction of an upper end of the first catheter; and a blocking part coupled to an upper outer side surface of the second catheter, capable of adjusting a position according to a movement of the upper end of the first catheter, and obliquely penetrating and blocking the orifice formed by the incomplete closing of the tricuspid valve.

## Description

### CROSS REFERENCE TO RELATED APPLICATION

The present application claims priority to Korean Patent Application No. 10-2019-0006359, filed January 17, 2019, and Korean Patent Application No. 10-2019-0055911, filed May 13, 2019, the entire contents of which are respectively incorporated herein for all purposes by this reference.

### BACKGROUND OF THE INVENTION

### Field of the Invention

The present invention relates to a position-adjustable device for treatment of tricuspid regurgitation. More particularly, the present invention relates to a position-adjustable device for treatment of tricuspid regurgitation, wherein the device is used to verify whether heart failure of the right ventricle may occur when tricuspid regurgitation is treated by surgery or another permanent treatment, and wherein the device is inserted to penetrate obliquely into a tricuspid valve of a patient so that the position of the inserted device may be adjusted to accurately block an orifice caused by the incomplete closing of the tricuspid valve depending on different patients, and may be removed after a certain time.

### Description of the Related Art

The heart is composed of four hollow chambers: the left atrium, the left ventricle, the right atrium, and the right ventricle, and are connected to the aorta, the vena cava, the pulmonary artery, and the pulmonary vein. There are valves between the ventricles and the atria. The valve between the left atrium and the left ventricle is called the mitral valve, and the valve between the right atrium and the right ventricle is called the tricuspid valve.

The heart repeats contraction and relaxation to enable blood to circulate. In the systolic phase of the heart, blood in the heart moves to blood vessels. The blood in the right ventricle flows to the pulmonary artery, and the blood in the left ventricle flows to the aorta.

However, when a valve is not working properly, blood backflow occurs, thereby causing the blood that must flow into blood vessels to flow back to the atrium during the heart contraction.

Tricuspid regurgitation is also known as tricuspid valve insufficiency. Tricuspid regurgitation is a lesion of the tricuspid valve, and refers to a condition in which the tricuspid valve does not close completely when the valve should be closed, thereby resulting in an orifice. Therefore, some blood, which should exit from the right ventricle into the pulmonary artery during the right ventricular contraction, is allowed to flow back through the orifice into the right atrium.

In conventional methods for treating tricuspid regurgitation, there is a method of surgical remedy by opening the patient's chest and dissecting the heart. This is an extremely invasive method. Therefore, in order to improve the conventional method, the devices for the treatment of tricuspid regurgitation have been devised in United States Patent Nos. 8486136 B2, 7854762 B2, and 9474605 B2. Those devices for the treatment of tricuspid regurgitation are inserted into the superior vena cava, tricuspid valve, and right ventricle in sequence, so as to block an orifice of the tricuspid valve to treat the tricuspid regurgitation. In the devices for the treatment of tricuspid regurgitation, an anchor installed at one end thereof is fixed to a ventricle, and the other end thereof is fixed to the outside of the heart through the superior vein cava. Accordingly, the blocking part of the related patents has a disadvantage in that the blocking part is placed in an unstable state of suspending in the heart, through the center of the orifice of tricuspid valve in a longitudinal orientation.

In addition, the above mentioned devices for the treatment of tricuspid regurgitation are provided to be permanently inserted into the patient's body in order to treat the tricuspid regurgitation. When treating tricuspid regurgitation using a permanent device for the treatment thereof, the sudden increase of blood flow from the right ventricle to the pulmonary artery may induce to overload of the right ventricle, thereby causing a problem of the right ventricular dysfunction symptoms.

Thus, before treating the tricuspid regurgitation by using a permanently inserted treatment device, the treatment device is required to be temporarily inserted into the body in order to identify in advance a problem occurring in a patient.

### Documents of Related Art

(Patent Document 1) US8486136 B2 (issued on July 16, 2013)
(Patent Document 2) US7854762 B2 (issued on December 21, 2010)
(Patent Document 3) US9474605 B2 (issued on October 25, 2016)

### SUMMARY OF THE INVENTION

The present invention is to provide a position-adjustable device for the treatment of tricuspid regurgitation, the device obliquely passing through the tricuspid valve and capable of being easily inserted through the inferior vena cava, the tricuspid valve, and the pulmonary artery in sequence to solve the above-mentioned problems.

In addition, the present invention is to provide a position-adjustable device for the treatment of tricuspid regurgitation, the device being capable of verifying whether overload of the right ventricle occurs by blocking an empty space (i.e., orifice) of the tricuspid valve for a certain time.

In addition, the present invention is to provide a position-adjustable device for the treatment of tricuspid regurgitation, the device being capable of more effectively blocking the orifice of the tricuspid valve by enabling the operator to adjust the blocking part to the orifice position of the tricuspid valve, which varies depending on different patients.

The objectives of the present invention are not limited to the above-mentioned objectives and other objectives that are not mentioned will be clearly understood by those skilled in the art from the following description.

In order to achieve the objectives, there is provided a position-adjustable device for the treatment of tricuspid regurgitation according to the preferred exemplary embodiments of the present invention, the device including: a first catheter provided with a first lumen; a second catheter inserted into the first lumen and provided with a second lumen into which a guidewire is inserted; an adjusting part coupled to lower sides of the first catheter and the second catheter, wherein the adjusting part is provided with a first direction-adjusting device adjusting a direction of an upper end of the first catheter; and a blocking part coupled to an upper outer side surface of the second catheter, capable of adjusting a position thereof according to a movement of the upper end of the first catheter, and obliquely penetrating and blocking an orifice formed by incomplete closing of a tricuspid valve.

On an inner wall, the first catheter may include: a plurality of lumens for first direction-adjusting wires, the lumens provided in parallel to the first lumen and having a closed upper end thereof; and a first lumen supporter coupled to an upper inner circumferential surface of the first catheter, the first lumen supporter provided with a protrusion closing upper ends of the plurality of lumens for the first direction-adjusting wires.

The first direction-adjusting device may include: a first left and right rotating gear installed therein and rotating left and right; a pair of first left and right driven gears installed on a left and a right of the first left and right rotating gear so as to perpendicularly engage with the first left and right rotating gear; and a plurality of first direction-adjusting wires having a lower end thereof coupled to the first left and right driven gears and having an upper end thereof coupled to the first catheter, wherein the first left and right driven gears may be rotated when the first left and right rotating gear is rotated left or right, so that an upper part of the first catheter may move to the left or to the right as the direction-adjusting wires are wound or loosened.

The first direction-adjusting device may include: a first left and right rotating gear installed therein and rotating left and right; a pair of first left and right driven gears installed on a left and a right of the first left and right rotating gear so as to perpendicularly engage with the first left and right rotating gear; a first forward and backward rotating gear installed in parallel and spaced apart from the first left and right rotating gear; a pair of first forward and backward driven gears installed so as to perpendicularly engage with the first forward and backward rotating gear at a front and a rear of the first forward and backward rotating gear; and a plurality of first direction-adjusting wires having a lower end thereof coupled to the first left and right driven gears or the first forward and backward driven gears and having an upper end thereof coupled to the first catheter, wherein the first left and right driven gears or the first forward and backward driven gears are rotated when the first left and right rotating gear or the first forward and backward rotating gear rotates, so that an upper part of the first catheter moves in one direction among forward, backward, leftward, and rightward as the direction-adjusting wires are wound or loosened.

The second catheter may be provided with a bending part curved at a predetermined angle at an upper one side thereof, wherein the bending part may be unfolded when the guidewire is inserted, and the bending part may be bent back to its original shape when the guidewire is removed.

The blocking part may further include: a supporting wire having both ends thereof coupled to a connecting tube; and a blocking membrane having one side thereof fixed to the connecting tube and supported by the supporting wire.

According to another exemplary embodiment, the blocking part may further include: a ring-shaped wire installed to enable a connecting tube to pass through and having a central axis thereof provided obliquely with respect to the connecting tube; and a blocking membrane connecting the connecting tube and the ring-shaped wire to each other.

The adjusting part may include a hemostatic valve installed at a lower end of the adjusting part and covering the lower end of the adjusting part, wherein the hemostatic valve may prevent an outflow of blood to the lower end of the adjusting part. In addition, the adjusting part may include a blood coagulation prevention port provided at a position on one side of the adjusting part and communicating with the adjusting part, wherein the blood coagulation prevention port may inject glucose, blood, or physiological saline to prevent the blood inserted into the position-adjustable device for the treatment of the tricuspid regurgitation from coagulating, and to induce the blood to move back into a patient's body.

According to another preferred exemplary embodiments of the present invention, a position-adjustable device for treatment of tricuspid regurgitation includes: a first catheter provided with a first lumen; a second catheter inserted into the first lumen and provided with a second lumen into which a guidewire is inserted; an adjusting part coupled to lower sides of the first catheter and the second catheter, wherein the adjusting part is provided with a first direction-adjusting device adjusting a direction of an upper end of the first catheter and a second direction-adjusting device adjusting a direction of an upper end of the second catheter; and a blocking part coupled to an upper side surface of the second catheter and obliquely penetrating and blocking an orifice formed by an incomplete closing of a tricuspid valve.

The device for the treatment of tricuspid regurgitation for pulmonary artery insertion according to the present invention may be simply inserted through the femoral vein, the inferior vena cava, the tricuspid valve, and the pulmonary artery in sequence.

In addition, before the device for the treatment of tricuspid regurgitation is permanently inserted, it is possible to verify whether the right ventricle is overloaded by temporarily inserting the device for the treatment of tricuspid regurgitation into the patient's body.

In addition, when abnormal symptoms due to the right ventricular overload of the patient are verified, the device may be easily removed so as to restore the blood pressure in the right ventricle.

In addition, the device position may be adjusted in accordance with the orifice of the tricuspid valve, the position varying depending on different patients, so that a patient-specific treatment may be performed.

### BRIEF DESCRIPTION OF THE DRAWINGS

FIG. 1 is a perspective view of a position-adjustable device for the treatment of tricuspid regurgitation according to a preferred exemplary embodiment of the present invention.
FIG. 2 is a cross sectional view of a first direction-adjusting device as an exemplary embodiment of the first direction-adjusting device according to the preferred exemplary embodiment of the present invention.
FIGS. 3A and 3B are views illustrating an exemplary embodiment of a first catheter according to the preferred exemplary embodiment of the present invention, in which FIG. 3A is a longitudinal cross-sectional view of the first catheter, and FIG. 3B is a transversal cross-sectional view of the first catheter.
FIG. 4 is a cross-sectional view of the first direction-adjusting device as another exemplary embodiment of the first direction-adjusting device according to the preferred exemplary embodiment of the present invention.
FIGS. 5A and 5B are views illustrating another exemplary embodiment of the first catheter according to the preferred exemplary embodiment of the present invention, in which FIG. 5A is a longitudinal cross-sectional view of the first catheter, and FIG. 5B is a transversal cross-sectional view of the first catheter.
FIG. 6 is a perspective view of the position-adjustable device for the treatment of tricuspid regurgitation according to another preferred exemplary embodiment of the present invention.
FIG. 7 is a cross-sectional view of a second direction-adjusting device as an exemplary embodiment of the second direction-adjusting device according to another preferred exemplary embodiment of the present invention.
FIGS. 8A and 8B are views illustrating an exemplary embodiment of a second catheter according to another preferred exemplary embodiment of the present invention, in which FIG. 8A is a longitudinal cross-sectional view of the second catheter, and FIG. 8B is a transversal cross-sectional view of the second catheter.
FIG. 9 is a cross-sectional view of the second direction-adjusting device according to another exemplary embodiment of the second direction-adjusting device according to another preferred exemplary embodiment of the present invention.
FIGS. 10A and 10B are views illustrating another exemplary embodiment of the second catheter according to another preferred exemplary embodiment of the present invention, in which FIG. 10A is a longitudinal cross-sectional view of the second catheter, and FIG. 10B is a transversal cross-sectional view of the second catheter.
FIG. 11 is a perspective view of the position-adjustable device for the treatment of tricuspid regurgitation according to another preferred exemplary embodiment of the present invention.
FIG. 12 is a partial perspective view showing another blocking part installed in the position-adjustable device for the treatment of tricuspid regurgitation according to the present invention.

### DETAILED DESCRIPTION OF THE INVENTION

Hereinafter, exemplary embodiments of the present invention will be described in detail with reference to the accompanying drawings.

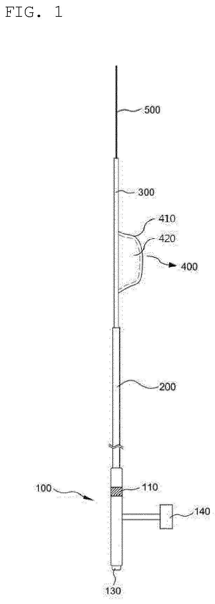

FIG. 1 is a perspective view of a position-adjustable device for the treatment of tricuspid regurgitation according to a preferred exemplary embodiment of the present invention.

FIG. 1 is a perspective view of a position-adjustable device for the treatment of tricuspid regurgitation according to a preferred exemplary embodiment of the present invention. Referring to FIG. 1, the position-adjustable device for the treatment of tricuspid regurgitation according to the present invention includes an adjusting part 100, a first catheter 200, a second catheter 300, and a blocking part 400.

The first catheter 200 has a cylindrical long tube shape like a general catheter and has a first lumen 210 provided therein. The lower part of the first catheter 200 is coupled to the adjusting part 100.

The first catheter 200 is composed of a flexible material, and may be a synthetic resin material such as a rubber material or a soft plastic material, and uses a material having high flexibility and excellent resilience to be movable according to the heartbeat.

The first lumen 210 is a cavity in which a wire or a tube may be inserted into the first catheter 200, and is provided by adjusting the diameter thereof according to an object inserted thereinto. The central axis of the first lumen 210 is the same as the central axis of the first catheter 200, and the first lumen 210 is preferably provided the same as the outer shape of the first catheter 200. For example, when the first catheter 200 is cylindrical, the first lumen 210 is also provided in cylindrical shape. When the first catheter 200 is a rectangular cylinder, the first lumen 210 is also provided in rectangular cylinder shape.

According to a preferred exemplary embodiment of the present invention, the diameter of the first lumen 210 is greater than the diameter of the second catheter 300 so that the second catheter 300 is inserted into the first lumen 210.

The second catheter 300 has a lower part thereof inserted into the first lumen 210 and a lower end thereof is coupled to the adjusting part 100. More particularly, the lower part of the second catheter 300 is inserted into the first lumen 210, and an upper part thereof protrudes out of the first lumen 210.

The second catheter 300 has a cylindrical long tube shape the same as the first catheter 200, and a second lumen 310 is provided therein. The second catheter 300 is composed of a flexible material, and may be a synthetic resin material such as a rubber material or a soft plastic material, and uses a material having high flexibility and excellent resilience to be movable according to the heartbeat.

The second lumen 310 is a cavity in which a wire or a tube may be inserted into the second catheter 300, and is provided by adjusting the diameter thereof according to an object inserted thereinto. The central axis of the second lumen 310 is the same as the central axis of the second catheter 300, and the second lumen 310 is preferably provided the same as the outer shape of the second catheter 300.

In addition, according to the preferred exemplary embodiment of the present invention, the diameter of the second lumen 310 is provided greater than the diameter of a guidewire 500 so that the guidewire 500 is inserted into the second lumen 310.

In this case, the guidewire 500 may be a single wire or a form made by twisting a plurality of wires, and synthetic resin such as nylon, metal (i.e., stainless steel, nylon coating on metal, etc.) wire, and the like may be used. In addition, it is desirable to have a diameter of about 0.014".

In this case, the second catheter 300 may include a bending part (not shown) bent to one side of an upper part thereof at a predetermined angle in order for the blocking part 400 to more effectively block the orifice of the tricuspid valve.

The second catheter 300 including the bending part (not shown) may move into the patient's body.

Alternatively, it is apparent that the bending part (not shown) may be unfolded when the guidewire 500 is inserted into the second catheter 300, and may be bent back to its original shape when the guidewire 500 is removed, so as to facilitate the movement of the catheter in the body.

The adjusting part 100 is positioned outside the patient's body to be adjusted by the hands of the operator. According to the preferred exemplary embodiment of the present invention, the adjusting part 100 includes a first direction-adjusting device 110. The adjusting part 100 has a shape that is easy for the operator to hold by hand. The adjusting part 100 may be provided with various shapes, such as cylinder, a cylinder having a curve, and the like, and has an interior with an empty space.

The first direction-adjusting device 110 is to adjust the movement of an upper end of the first catheter 200, is installed on one side of the adjusting part 100, and more preferably, is installed on one side of the adjusting part 100 horizontally across the adjusting part 100.

When the first direction-adjusting device 110 includes one rotating gear, the upper end of the first catheter 200 may move in two directions (forward and backward or left and right) . When the first direction-adjusting device 110 includes two rotating gears, the upper end of the first catheter 200 may move in four directions (forward, backward, left, and right).

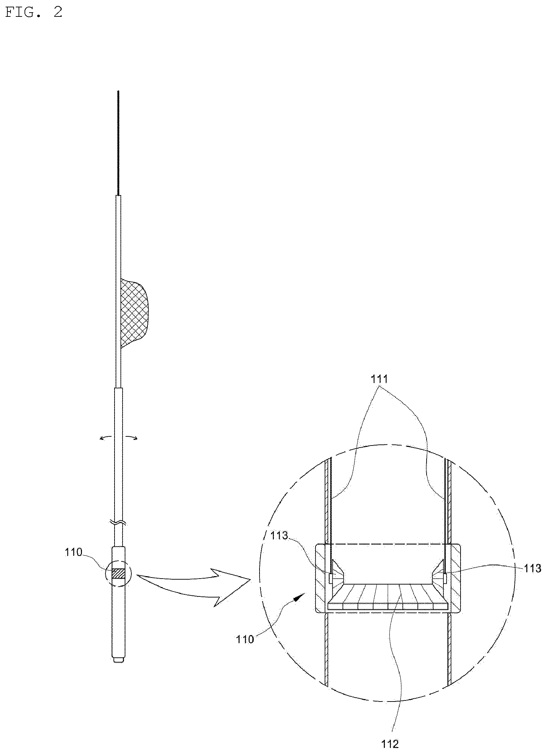

According to the exemplary embodiment of the first direction-adjusting device 110, the first catheter 200 also has various exemplary embodiments. Detailed description thereof will be described with reference to FIGS. 2 to 5.

The adjusting part 100 further includes a hemostatic valve 130 coupled to the lower end thereof. The hemostatic valve 130 blocks the lower end of the adjusting part 100 so that the blood flowing into the adjusting part 100 from the first catheter 200 and the second catheter 300 is prevented from leaking to the lower end of the adjusting part 100. The hemostatic valve 130 is preferably made of a silicon material, but is not limited thereto.

In addition, the adjusting part 100 of the position-adjustable device for the treatment of tricuspid regurgitation according to the preferred exemplary embodiment of the present invention is further includes a blood coagulation prevention port 140 coupled to and communicated with one side of the adjusting part 100. When inserting the position-adjustable device for the treatment of tricuspid regurgitation according to the present invention into the patient's body to temporarily block the orifice of the tricuspid valve, one side of the device is inserted into the body for as short as a few hours or as long as two to three days.

In this case, the blood coagulation prevention port 140 injects glucose, blood, or physiological saline to push the blood flowing into the adjusting part 100 to induce the blood to move back into the patient's body, whereby the blood flowing into the adjusting part 100 is not coagulated even when the device for the treatment of tricuspid regurgitation is maintained in a state of being inserted into the body.

FIG. 2 is a cross sectional view of the first direction-adjusting device as an exemplary embodiment of the first direction-adjusting device according to the preferred exemplary embodiment of the present invention.

Referring to FIG. 2 to describe an exemplary embodiment of the first direction-adjusting device 110, the first direction-adjusting device 110 includes a first left and right rotating gear 112, a first left and right driven gear 113, and a first direction-adjusting wire 111.

The first left and right rotating gear 112 is installed inside the first direction-adjusting device 110, and rotates in the same direction as the user rotates the first direction-adjusting device 110 to the left or right.

Each of a pair of first left and right driven gears 113 are respectively installed on the left and right sides of the first left and right rotating gear 112 so as to perpendicularly engage with the first left and right rotating gear 112. The first left and right driven gears 113 are driven as the first left and right rotating gear 112 is rotated. The first left and right rotating gear 112 and the first left and right driven gear 113 are operated in the same way as a bevel gear, and correspond to the ring gear and pinion gear of the bevel gear.

The pair of first left and right driven gears 113 are rotated with each other in opposite directions. For example, when the first left and right rotating gear 112 is rotated to the left, one of the first left and right driven gears 113 is driven in the clockwise direction and the other of the first left and right driven gear 113 is driven in the counterclockwise direction.

The first direction-adjusting wire 111 has a lower end thereof coupled to the first left and right driven gear 113 and an upper end thereof coupled to the first catheter 200. Each of the first direction-adjusting wires 111 is respectively coupled to each of the pair of the first left and right driven gear 113, one by one, and synthetic resin such as nylon, metal (stainless steel, nylon coating on the metal, etc.) wire, and the like may be used. It is apparent that the first direction-adjusting wire 111 may be a single wire, and may also be a form of wire made by twisting a plurality of wires.

As the first direction-adjusting wire 111 is wound or loosened on one side of the first left and right driven gear 113, the first catheter 200 is pulled or pushed to adjust the direction of the first catheter 200.

More particularly, when the first left and right rotating gear 112 is rotated to the left direction by turning the first direction-adjusting device 110 to the left direction, the first left and right driven gear 113, engaged with the left side of the first left and right rotating gear 112, is driven to the forward direction. Accordingly, the first direction-adjusting wire 111 is wound around the first left and right driven gear 113. Therefore, since the upper end of the first catheter 200 is pulled to the left side, the upper end of the first catheter 200 moves to the left.

Whereas, when the first left and right rotating gear 112 is rotated to the right direction by rotating the first direction-adjusting device 110 to the right direction, the first left and right driven gear 113, engaged with the left side of the first left and right rotating gear 112, is driven to the backward direction, and thus the first direction-adjusting wire 111 wound around the first left and right driven gear 113 is loosened. At the same time, the first left and right driven gear 113, engaged with the right side of the first left and right rotating gear 112, is driven to the forward direction, and thus the first direction-adjusting wire 111 is wound around the first left and right driven gear 113. Therefore, since the upper end of the first catheter 200 is pulled to the right side, the upper end of the first catheter 200 moves to the right side.

Therefore, the first catheter 200 may be moved to the left side or the right side.

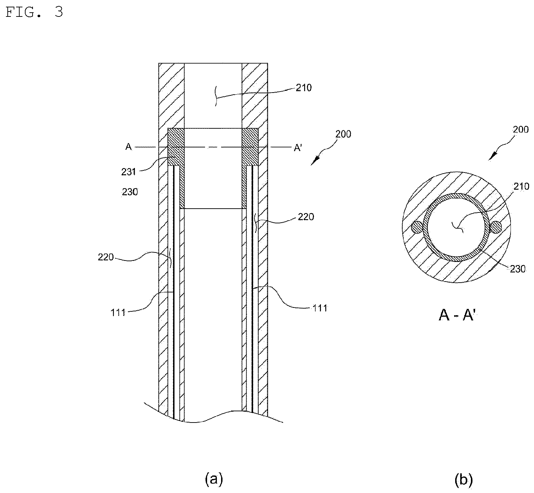

FIGS. 3A and 3B are views illustrating an exemplary embodiment of the first catheter according to the preferred exemplary embodiment of the present invention, in which FIG. 3A is a longitudinal cross-sectional view of the first catheter, and FIG. 3B is a transversal cross-sectional view of the first catheter.

The first catheter 200 includes a plurality of lumens 220 for the first direction-adjusting wire on the inner wall of the first catheter 200. The lumen 220 for the first direction-adjusting wire is provided in parallel to the first lumen 210. The first catheter 200 includes two lumens 220 for the first direction-adjusting wire provided on the left and right of the first lumen 210 centered around the first lumen 210 on the cross section of the first catheter 200.

The diameter of the plurality of lumens 220 for the first direction-adjusting wire is smaller than the diameter of the first lumen 210, and is provided to have a diameter to which the first direction-adjusting wire 111 may be inserted.

In addition, the lumen 220 for the first direction-adjusting wire is not provided up to the position at the upper end of the first catheter 200, but is provided up to the position where the first lumen supporter 230 is coupled to the upper inner circumferential surface of the first catheter 200.

The first lumen supporter 230 is provided at a position along an inner circumferential surface of the first catheter 200 in a quadrangular band shape and is coupled to an upper inner circumferential surface of the first catheter 200, and come in contact with and coupled to one side of the plurality of lumens 200 for the first direction-adjusting wire. In addition, the first lumen supporter 230 is provided to have a plurality of protrusions 231 closing the upper ends of the plurality of lumens 220 for the first direction-adjusting wire.

Accordingly, on the lower side of the first lumen supporter 230 as a baseline, the first catheter 200 is a multi-lumen catheter having a plurality of lumens. Also, on the upper side of the first lumen supporter 230 as the baseline, the first catheter 200 is a single-lumen catheter having one lumen.

In this case, each of the first direction-adjusting wires 111 is inserted into a plurality of lumens 230 for the first direction-adjusting wire, respectively. More particularly, the first direction-adjusting wire 111 is inserted into the lumen 230 for the first direction-adjusting wire, the upper end of the wire is fixed to the protrusion 231 closing the upper end of the lumen 230 for the first direction-adjusting wire, and the lower end of the wire is coupled to the first left and right driven gear 113 of the first direction-adjusting device 110.

Accordingly, as the first direction control wire 111 is wound or loosened around the first left and right driven gear 113, the upper part of the first catheter 200 is pulled or pushed to move the first catheter 200 in one direction.

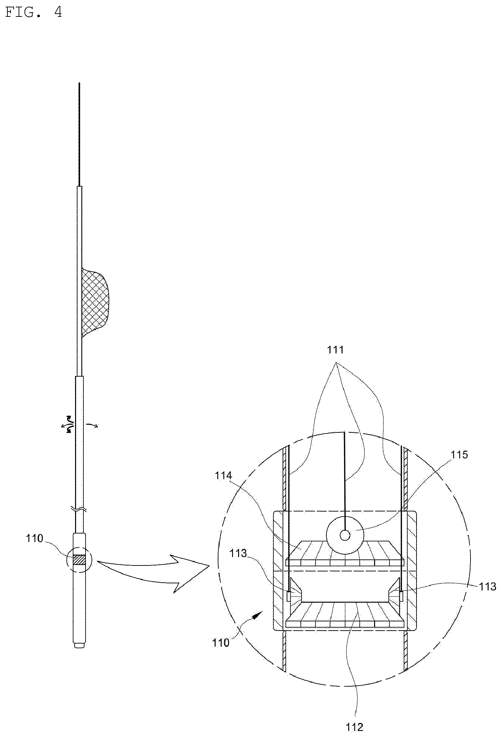

FIG. 4 is a cross-sectional view of the first direction-adjusting device as another exemplary embodiment of the first direction-adjusting device according to the preferred exemplary embodiment of the present invention.

Another exemplary embodiment of the first direction-adjusting device 110 according to FIG. 4 includes the first left and right rotating gear 112, the first left and right driven gear 113, a first forward and backward rotating gear 114, a first forward and backward driven gear 115, and the first direction-adjusting wire 111.

The first direction-adjusting device 110 is divided into two zones (i.e., partitions), and the first left and right rotating gear 112 and the first left and right driven gear 113 are installed inside one zone, and the first forward and backward rotating gear 114 and the first forward and backward driven gear 115 are installed inside the other zone. The first direction-adjusting device 110 divided into the two zones is capable of rotating separately.

The first left and right rotating gear 112, the first left and right driven gear 113, and the first direction-adjusting wire 111 are operated in the same way as described above with reference to FIG. 2.

The first forward and backward rotating gear 114 is installed in parallel to the first left and right rotating gear 112 inside the first direction-adjusting device 110, and is also rotated in the same direction as the user rotates the first direction-adjusting device 110 to the left or right.

A pair of first forward and backward driven gears 115 are respectively installed at the front and the rear of the first forward and backward rotating gear 114 so as to perpendicularly engage with the first forward and backward rotating gear 114. The first forward and backward driven gears 115 are driven as the first forward and backward rotating gear 114 is rotated. The first forward and backward rotating gear 114 and the first forward and backward driven gear 115 are operated in the same way as a bevel gear, and correspond to the ring gear and pinion gear of the bevel gear.

More particularly, the pair of first forward and backward driven gears 115 are rotated in opposite directions. For example, when the first forward and backward rotating gear 114 is rotated to the left, one of the first forward and backward driven gears 115 are driven in the clockwise direction and the other of the first forward and backward driven gears 115 is driven in the counterclockwise direction.

The first direction-adjusting wire 111 is the same as that wire coupled to the first left and right driven gear 113, the bottom end thereof is coupled to the first forward and backward driven gear 115, and the upper end thereof is coupled to the first catheter 200.

As the first direction-adjusting wire 111 is wound or loosened on one side of the first forward and backward driven gear 115, the first catheter 200 is pulled or pushed to adjust the direction of the first catheter 200.

More particularly, when the first forward and backward rotating gear 114 is rotated to the left direction by turning the first direction-adjusting device 110 to the left direction, the first forward and backward driven gear 115, engaged with the front side of the first forward and backward rotating gear 114, is driven to the left direction. Accordingly, the first direction-adjusting wire 111 is wound around the first forward and backward driven gear 115. Therefore, since the upper end of the first catheter 200 is pulled to the front side, the upper end of the first catheter 200 moves to the front side.

Whereas, when the first forward and backward rotating gear 114 is rotated to the right direction by rotating the first direction-adjusting device 110 to the right direction, the first forward and backward driven gear 115, engaged with the front side of the first forward and backward rotating gear 114, is driven to the right direction, and thus the first direction-adjusting wire 111, wound around the first forward and backward driven gear 115, is loosened. At the same time, the first forward and backward driven gear 115, engaged with the right side of the first forward and backward rotating gear 114, is driven to the left direction, and thus the first direction-adjusting wire 111 is wound around the first forward and backward driven gear 115. Therefore, since the upper end of the first catheter 200 is pulled to the rear side, the upper end of the first catheter 200 moves to the rear side.

Therefore, the upper end of the first catheter 200 may be adjusted in four directions: front side, rear side, left side, or right side.

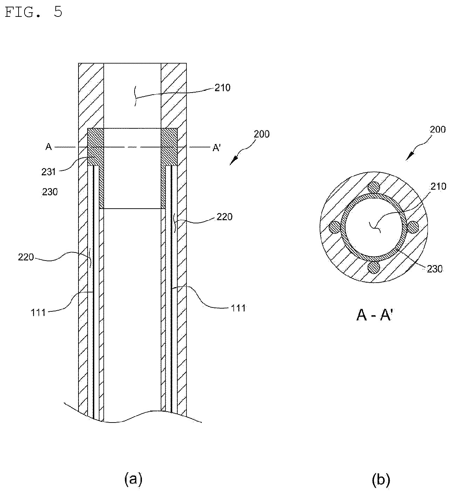

FIGS. 5A and 5B are views illustrating another exemplary embodiment of the first catheter according to the preferred exemplary embodiment of the present invention, in which FIG. 5A is a longitudinal cross-sectional view of the first catheter, FIG. 5B is a transversal cross-sectional view of the first catheter.

The first catheter 200 includes a plurality of lumens 220 for the first direction-adjusting wire on the inner wall of the first catheter 200. The lumen 220 for the first direction-adjusting wire is provided in parallel to the first lumen 210. The first catheter 200 includes four lumens 220 for the first direction-adjusting wire installed spaced apart from each other at the top, bottom, left, and right of the first lumen 210 centered around the first lumen 210 on the cross section of the first catheter 200.

The diameter of the plurality of lumens 220 for the first direction-adjusting wire is smaller than the diameter of the first lumen 210, and is provided to have a diameter to which the first direction-adjusting wire 111 may be inserted.

In addition, the lumen 220 for the first direction-adjusting wire is not provided up to the position at the upper end of the first catheter 200, and is provided up to the position where the first lumen supporter 230 is coupled to the upper inner circumferential surface of the first catheter 200.

The first lumen supporter 230 is provided along an inner circumferential surface of the first catheter 200 in a quadrangular band shape and is coupled to an upper inner circumferential surface of the first catheter 200, and come in contact with and coupled to one side of the plurality of lumens 220 for the first direction-adjusting wire. In addition, the first lumen supporter 230 is provided to have protrusions 231 closing the upper end of the plurality of lumens 220 for the first direction-adjusting wire.

Accordingly, on the lower side of the first lumen supporter 230 as a baseline, the first catheter 200 is a multi-lumen catheter having a plurality of lumens. Also, on the upper side of the first lumen supporter 230 as the baseline, the first catheter 200 is a single-lumen catheter having one lumen.

In this case, each of the first direction-adjusting wires 111 is respectively inserted into the plurality of lumens 230 for the first direction-adjusting wire. More particularly, the first direction-adjusting wire 111 is inserted into the lumen 230 for the first direction-adjusting wire, the upper end of the wire is fixed to the protrusion 231 closing the upper end of the lumen 230 for the first direction-adjusting wire, and the lower end of the wire is coupled to the first left and right driven gear 113 and first forward and backward driven gear 115 of the first direction-adjusting device 110.

Accordingly, as the first direction control wire 111 is wound or loosened around the first left and right driven gear 113 and the first forward and backward driven gear 115, the upper part of the first catheter 200 is pulled or pushed to move the first catheter 200 in one direction.

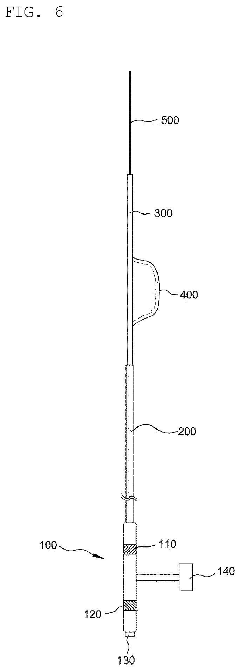

FIG. 6 is a perspective view of the position-adjustable device for the treatment of tricuspid regurgitation according to another preferred exemplary embodiment of the present invention.

Referring to FIG. 6, the position-adjustable device for the treatment of tricuspid regurgitation according to another embodiment of the present invention includes the adjusting part 100, the first catheter 200, the second catheter 300, and the blocking part 400. The adjusting part 100 includes the first direction-adjusting device 110 and the second direction-adjusting device 120.

The first catheter 200, the blocking part 400, and the first direction-adjusting device 110 are the same as described above in FIGS. 1 to 5, and the first direction-adjusting device 110 and the first catheter 200 may be provided in the various modes of the exemplary embodiments as described with reference to FIGS. 2 to 5.

In the adjusting part 100 according to another preferred exemplary embodiment of the present invention, the first direction-adjusting device 110 and the second direction-adjusting device 120 are installed to be spaced apart from each other. On the upper side of the adjusting part 100, the first direction-adjusting device 110 is installed, and the second direction-adjusting device 120 is preferably installed on the lower side of the adjusting part 100, but is not limited thereto.

The second direction-adjusting device 120 is to adjust the movement of the upper end of the second catheter 300. When the second direction-adjusting device 120 includes one rotating gear, the upper end of the second catheter 300 may move in two directions (forward and backward or left and right), and when the second direction-adjusting device 120 includes two rotating gears, the upper end of the second catheter 300 may move in four directions (forward, backward, left, and right).

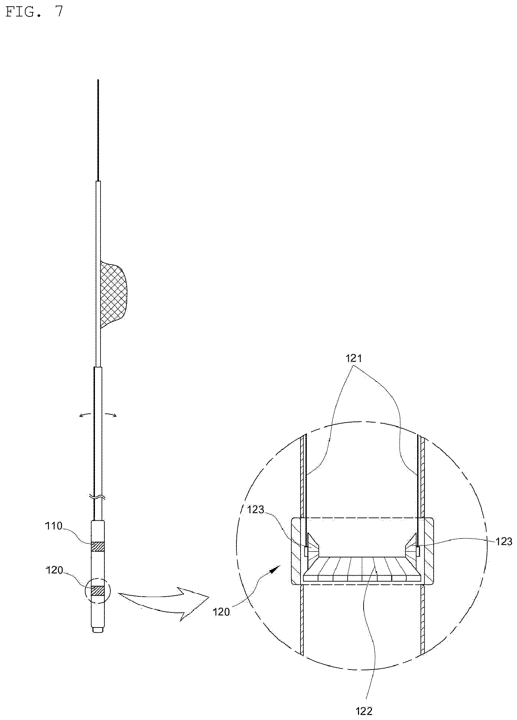

According to the exemplary embodiment of the second direction-adjusting device 120, the second catheter 300 also has various exemplary embodiments. Detailed description thereof will be described with reference to FIGS. 7 to 10.

FIG. 7 is a cross-sectional view of the second direction-adjusting device as the exemplary embodiment of the second direction-adjusting device according to another preferred exemplary embodiment of the present invention.

Referring to FIG. 7 to describe the exemplary embodiment of the second direction-adjusting device 120, the second direction-adjusting device 120 includes a second left and right rotating gear 122, a second left and right driven gear 123, and a second direction-adjusting wire 121.

The second left and right rotating gear 122 is installed inside the second direction-adjusting device 120, and rotates in the same direction as the user rotates the second direction-adjusting device 120 to the left or right direction.

Each of the pair of second left and right driven gears 123 are respectively installed on the left and right sides of the second left and right rotating gear 122 so as to perpendicularly engage with the second left and right rotating gear 122. The second left and right driven gear 123 is driven as the second left and right rotating gear 122 is rotated. The second left and right rotating gear 122 and the second left and right driven gear 123 are operated in the same way as a bevel gear, and correspond to the ring gear and pinion gear of the bevel gear.

The pair of second left and right driven gears 123 are rotated with each other in opposite directions. For example, when the second left and right rotating gear 122 is rotated to the left, one of the second left and right driven gears 123 is driven in the clockwise direction, and the other of the second left and right driven gears 123 is driven in the counterclockwise direction.

The second direction-adjusting wire 121 has a lower end thereof coupled to the second left and right driven gear 123 and an upper end thereof coupled to the second catheter 300. Each of the second direction-adjusting wires 121 is respectively coupled to each of the pair of the second left and right driven gears 123, one by one, and may be made of synthetic resin such as nylon, metal (stainless steel, nylon coating on metal, etc.), and the like. It is apparent that the second direction-adjusting wire 121 may be a single wire, and may also be a form of wire made by twisting a plurality of wires.

As the second direction-adjusting wire 121 is wound or loosened on one side of the second left and right driven gear 123, the second catheter 300 is pulled or pushed to adjust the direction of the second catheter 300.

More particularly, when the second left and right rotating gear 122 is rotated to the left direction by turning the second direction-adjusting device 120 to the left direction, the second left and right driven gear 123, engaged with the left side of the second left and right rotating gear 122, is driven to the forward direction. Accordingly, the second direction-adjusting wire 121 is wound around the second left and right driven gear 123. Therefore, since the upper end of the second catheter 300 is pulled to the left side, the upper end of the second catheter 300 moves to the left side.

Whereas, when the second left and right rotating gear 122 is rotated to the right direction by rotating the second direction-adjusting device 120 to the right direction, the second left and right driven gear 123, engaged with the left side of the second left and right rotating gear 122, is driven to the backward direction, and thus the second direction-adjusting wire 121, wound around the second left and right driven gear 123, is loosened. At the same time, the second left and right driven gear 123, engaged with the right side of the second left and right rotating gear 122, is driven to the forward direction, and thus the second direction-adjusting wire 121 is wound around the second left and right driven gear 123. Therefore, since the upper end of the second catheter 300 is pulled to the right side, the upper end of the second catheter 300 moves to the right side.

Thus, the second catheter 300 may be moved to left side or the right side.

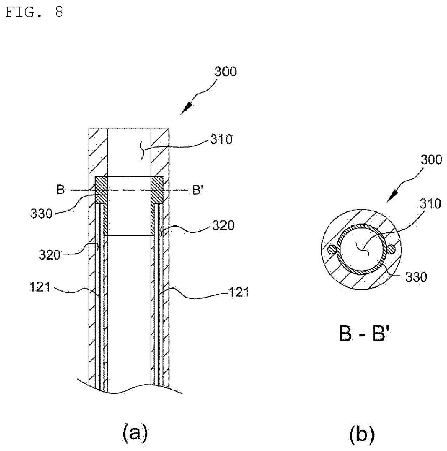

FIGS. 8A and 8B are views illustrating an exemplary embodiment of the second catheter according to another preferred exemplary embodiment of the present invention, in which FIG. 8A is a longitudinal cross-sectional view of the second catheter, and FIG. 8B is a transversal cross-sectional view of the second catheter.

The second catheter 300 includes a plurality of lumens 220 for the first direction-adjusting wire on the inner wall of the second catheter 300. The lumen 320 for the second direction-adjusting wire is provided in parallel to the second lumen 310. The second catheter 300 includes two lumens 320 for the second direction-adjusting wire provided on the left and right sides of the second lumen 310 centered around the second lumen 310 on the cross section of the second catheter 300.

The diameter of the plurality of lumens 320 for the second direction-adjusting wire is smaller than the diameter of the second lumen 310, and is provided to have a diameter to which the second direction-adjusting wire 121 may be inserted.

In addition, the lumen 320 for the second direction-adjusting wire is not provided up to the position at the upper end of the second catheter 300, and is provided up to the position where the second lumen supporter 330 is coupled to the upper inner circumferential surface of the second catheter 300.

The second lumen supporter 330 is provided along the inner circumferential surface of the second catheter 300 in a quadrangular band shape and is coupled to the upper inner circumferential surface of the second catheter 300, and come in contact with and coupled to one side of the plurality of lumens 320 for the second direction-adjusting wire. In addition, the second lumen supporter 330 is provided to have a plurality of protrusions 331 closing the upper ends of the plurality of lumens 320 for the second direction-adjusting wire.

Accordingly, on the lower side of the second lumen supporter 330 as a baseline, the second catheter 300 is a multi-lumen catheter having a plurality of lumens. Also, on the upper side of the second lumen supporter 330 as the baseline, the second catheter 300 is a single-lumen catheter having one lumen.

In this case, each of the second direction-adjusting wires 121 is respectively inserted into the plurality of lumens 320 for the second direction-adjusting wire. More particularly, the second direction-adjusting wire 121 is inserted into the lumen 320 for the second direction-adjusting wire, the upper end of the wire is coupled to the protrusion 331 closing the upper end of the lumen 320 for the second direction-adjusting wire, and the lower end of the wire is coupled to the second left and right driven gear 123 of the second direction-adjusting device 120.

Therefore, as the second direction-adjusting wire 121 is wound or loosened on the second left and right driven gear 123, the second catheter 300 is pulled or pushed in one direction to move the second catheter 300.

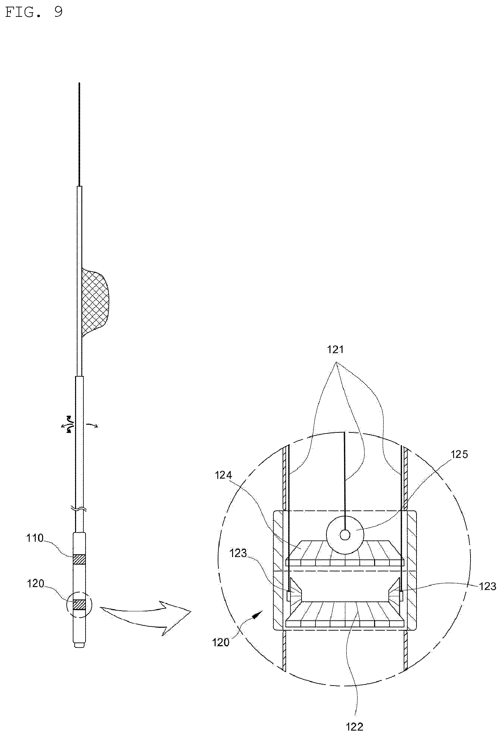

FIG. 9 is a cross-sectional view of the second direction-adjusting device as another exemplary embodiment of the second direction-adjusting device according to another preferred exemplary embodiment of the present invention.

Another exemplary embodiment of the second direction-adjusting device 120 according to FIG. 9 includes the second left and right rotating gear 122, the second left and right driven gear 123, a second forward and backward rotating gear 124, a second forward and backward driven gear 125, and the second direction-adjusting wire 121.

The second direction-adjusting device 120 is divided into two zones (i.e., partitions), and the second left and right rotating gear 122 and the second left and right driven gear 123 are installed inside one zone, and the second forward and backward rotating gear 124 and the second forward and backward driven gear 125 are installed inside the other zone. The second direction-adjusting device 120 divided into the two zones is capable of rotating separately.

The second left and right rotating gear 122, the second left and right driven gear 123, and the second direction-adjusting wire 121 are operated in the same way as described above with reference to FIG. 7.

The second forward and backward rotating gear 124 is installed in parallel to the second left and right rotating gear 122 in the second direction-adjusting device 120, and is also rotated in the same direction as the user rotates the second direction-adjusting device 120 to the left or right.

A pair of second front and rear driven gears 125 are respectively installed at the front and the rear of the second front and rear rotating gear 124 so as to perpendicularly engage with the second forward and backward rotating gear 124. The second forward and backward driven gear 125 is driven as the second forward and backward rotating gear 124 is rotated. The second forward and backward rotating gear 124 and the second forward and backward driven gear 125 are operated in the same way as a bevel gear, and correspond to the ring gear and pinion gear of the bevel gear.

More particularly, the pair of second forward and backward driven gears 125 are rotated in opposite directions. For example, when second forward and backward rotating gear 124 is rotated to the left, one of the second forward and backward driven gears 125 is driven in the clockwise direction, and the other of the second forward and backward driven gears 125 is driven in the counterclockwise direction.

The second direction-adjusting wire 121 is of the same shape and material as that coupled to the second left and right driven gear 123, the lower end of the wire is coupled to the second forward and backward driven gear 125, and the upper end of the wire is coupled to the second catheter 300.

As the second direction-adjusting wire 121 is wound or loosened on one side of the second forward and backward driven gear 125, the second catheter 300 is pulled or pushed to adjust the direction of the second catheter 300.

More particularly, when the second forward and backward rotating gear 124 is rotated to the left direction by turning the second direction-adjusting device 120 to the left direction, the second forward and backward driven gear 125, engaged with the front side of the second forward and backward rotating gear 124, is driven to the left direction. Accordingly, the second direction-adjusting wire 121 is wound around the second forward and backward driven gear 125. Therefore, since the upper end of the second catheter 300 is pulled to the front side, the upper end of the second catheter 300 moves to the front side.

Whereas, when the second forward and backward rotating gear 124 is rotated to the right direction by rotating the second direction-adjusting device 120 to the right direction, the second forward and backward driven gear 125, engaged with the front side of the second forward and backward rotating gear 124, is driven to the right direction, and thus the second direction-adjusting wire 121, wound around the second forward and backward driven gear 125, is loosened. At the same time, the second forward and backward driven gear 125, engaged with the right side of the second forward and backward rotating gear 124, is driven to the left direction, and thus the second direction-adjusting wire 121 is wound around one side of the second forward and backward driven gear 125. Therefore, since the upper end of the second catheter 300 is pulled to the rear side, the upper end of the first catheter 200 moves to the rear side.

Accordingly, the upper end of the second catheter 300 may be adjusted in four directions: front side, rear side, left side, or right side.

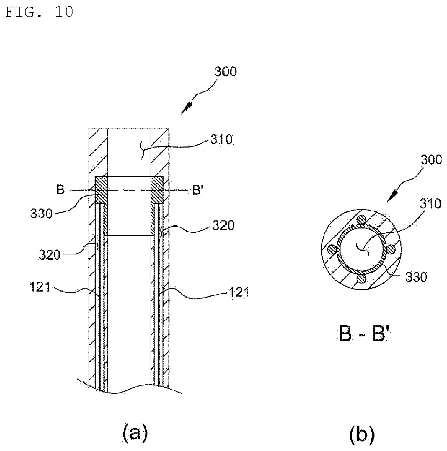

FIGS. 10A and 10B are views illustrating another exemplary embodiment of the second catheter according to another preferred exemplary embodiment of the present invention, in which FIG. 10 (a) is a longitudinal cross-sectional view of the second catheter, and FIG. 10 (b) is a transversal cross-sectional view of the second catheter.

The second catheter 300 includes a plurality of lumens 320 for the second direction-adjusting wire provided on the inner wall of the second catheter 300. The lumen 320 for the second direction-adjusting wire is provided in parallel to the second lumen 310. The second catheter 300 includes four lumens 320 for the second direction-adjusting wire provided spaced apart from each other at the top, bottom, left, and right of the second lumen 310 centered around the second lumen 310 on the cross section of the second catheter 300.

The diameter of the plurality of lumens 320 for the second direction-adjusting wire is smaller than the diameter of the second lumen 310, and is provided to have a diameter to which the second direction-adjusting wire 121 may be inserted.

In addition, the lumen 320 for the second direction-adjusting wire is not provided up to the position at the upper end of the second catheter 300, and is provided up to the position where the second lumen supporter 330 is coupled to the upper inner circumferential surface of the second catheter 300.

The second lumen supporter 330 is provided along the inner circumferential surface of the second catheter 300 in a quadrangular band shape and is coupled to the upper inner circumferential surface of the second catheter 300, and come in contact with and coupled to one side of the plurality of lumens 320 for the second direction-adjusting wire. In addition, the second lumen supporter 330 is provided to have a plurality of protrusions 331 closing the upper ends of the plurality of lumens 320 for the second direction-adjusting wire.

Accordingly, on the lower side of the second lumen supporter 330 as a baseline, the second catheter 300 is a multi-lumen catheter having a plurality of lumens. Also, on the upper side of the second lumen supporter 330 as the baseline, the second catheter 300 is a single-lumen catheter having one lumen.

In this case, each of the second direction-adjusting wires 121 is respectively inserted into the plurality of lumens 330 for the second direction control wire. More particularly, the second direction-adjusting wire 121 is inserted into the lumen 330 for the second direction-adjusting wire, the upper end of the wire is coupled to the protrusion 331 closing the upper end of the lumen 330 for the second direction-adjusting wire, and the lower end of the wire is coupled to the second left and right driven gear 123 and the second forward and backward driven gear 125 of the second direction-adjusting device 120.

Accordingly, as the second direction-adjusting wire 121 is wound or loosened by the second left and right driven gear 123 and the second forward and backward driven gear 125, the upper part of the second catheter 300 is pulled or pushed in one direction to move the second catheter 300.

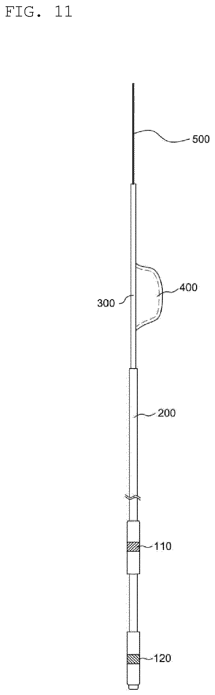

FIG. 11 is a perspective view of the position-adjustable device for the treatment of tricuspid regurgitation according to another preferred exemplary embodiment of the present invention.

In the position-adjustable device for the treatment of tricuspid regurgitation according to another preferred exemplary embodiment of the present invention, the first direction-adjusting device 110 and the second direction-adjusting device 120 are respectively installed, rather than installed together in the adjusting part 100.

As shown in FIG. 11, the first direction-adjusting device 110 is installed at a lower part of the first catheter 200, the second direction-adjusting device 120 is installed to be spaced apart from the lower end of the first direction-adjusting device 110, but is not limited thereto. It is apparent that the second direction-adjusting device 120 may be installed at an upper side of the first direction-adjusting device 110.

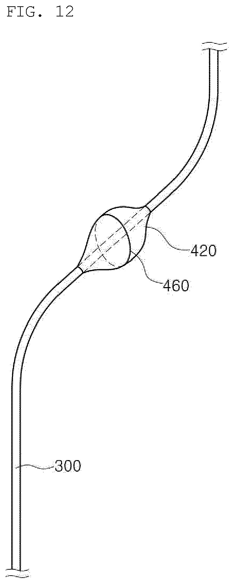

FIG. 12 is a partial perspective view showing another blocking part installed in the position-adjustable device for the treatment of tricuspid regurgitation according to the present invention.

Another blocking part 400 according to the present invention includes a blocking membrane 420 and a ring-shaped wire 460. The ring-shaped wire 460 is installed to penetrate the second catheter 300, the central axis of the ring-shaped wire is provided obliquely to the second catheter 300, and the blocking membrane 420 connects between the second catheter 300 and the ring-shaped wire 460. Since the ring-shaped wire 460 is provided to be inclined at the second catheter 300, the central axis of the ring-shaped wire is positioned in parallel to the tricuspid valve inclined at a certain angle, thereby being capable of effectively blocking the orifice of the tricuspid valve. The ring-shaped wire 460 may be made of synthetic resin such as nylon, metal (stainless steel, nylon coating on metal, etc.), and the like.

Hereinafter, a method for treating the tricuspid regurgitation will be described, the method using the position-adjustable device for the treatment of tricuspid regurgitation according to the present invention as described above.

First, the guidewire 500 is inserted into the inferior vena cava, the tricuspid valve, and the pulmonary artery in sequence. The position-adjustable device for the treatment of tricuspid regurgitation according to the present invention is inserted along the path where the guidewire 500 is inserted, and is inserted into a tube or catheter to facilitate the insertion thereof into the patient's body. When the blocking part 400 is positioned to obliquely penetrate the tricuspid valve, the blocking part 400 is unfolded by removing the tube or catheter.

Then, the first direction-adjusting device 110 and the second direction-adjusting device 120 are adjusted to match the position of the orifice generated due to the incomplete closing of the tricuspid valve, the position being different depending on the different patients. The upper part of the first catheter 200 is moved by adjusting the first direction-adjusting device 110, and the upper part of the second catheter 300 is moved by adjusting the second direction-adjusting device 120, whereby the position of the blocking part 400 is adjusted.

The adjusting part 100 is always positioned in the outside of the patient's body to be adjusted by the operator, and after a certain time, by checking the condition of the patient and pulling the adjusting part 100 to the lower side direction thereof, the device for the treatment of the present invention may be removed.

Therefore, the position-adjustable device for the treatment of tricuspid regurgitation according to the present invention may be easily inserted into or removed therefrom the patient's body, and the position of the blocking part may be adjusted according to the different patients, thereby being capable of performing a patient-specific treatment.

Although the exemplary embodiments of the present invention have been described above with reference to the accompanying drawings, it will be understood that those skilled in the art to which the present invention pertains may implement the present invention in other specific forms without departing from the technical spirit or essential features thereof. Therefore, the exemplary embodiments described above are to be understood in all respects as illustrative and not restrictive.

## Claims

1. A position-adjustable device for treatment of tricuspid regurgitation, the device comprising:
a first catheter provided with a first lumen;
a second catheter inserted into the first lumen and provided with a second lumen into which a guidewire is inserted;
an adjusting part coupled to lower sides of the first catheter and the second catheter, wherein the adjusting part is provided with a first direction-adjusting device adjusting a direction of an upper end of the first catheter; and
a blocking part coupled to an upper outer side surface of the second catheter, capable of adjusting a position thereof according to a movement of the upper end of the first catheter, and obliquely penetrating and blocking an orifice formed by incomplete closing of a tricuspid valve.

2. The device of claim 1, wherein, on an inner wall, the first catheter comprises:
a plurality of lumens for first direction-adjusting wires, the lumens provided in parallel to the first lumen and having a closed upper end thereof; and
a first lumen supporter coupled to an upper inner circumferential surface of the first catheter, the first lumen supporter provided with a protrusion closing upper ends of the plurality of lumens for the first direction-adjusting wires.

3. The device of claim 1, wherein the first direction-adjusting device comprises:
a first left and right rotating gear installed therein and rotating left and right;
a pair of first left and right driven gears installed on a left and a right of the first left and right rotating gear so as to perpendicularly engage with the first left and right rotating gear; and
a plurality of first direction-adjusting wires having a lower end thereof coupled to the first left and right driven gears and having an upper end thereof coupled to the first catheter, wherein the first left and right driven gears are rotated when the first left and right rotating gear is rotated left or right, so that an upper part of the first catheter moves to the left or to the right as the direction-adjusting wires are wound or loosened.

4. The device of claim 1, wherein the first direction-adjusting device comprises:
a first left and right rotating gear installed therein and rotating left and right;
a pair of first left and right driven gears installed on a left and a right of the first left and right rotating gear so as to perpendicularly engage with the first left and right rotating gear;
a first forward and backward rotating gear installed in parallel and spaced apart from the first left and right rotating gear;
a pair of first forward and backward driven gears installed so as to perpendicularly engage with the first forward and backward rotating gear at a front and a rear of the first forward and backward rotating gear; and
a plurality of first direction-adjusting wires having a lower end thereof coupled to the first left and right driven gears or the first forward and backward driven gears and having an upper end thereof coupled to the first catheter, wherein the first left and right driven gears or the first forward and backward driven gears are rotated when the first left and right rotating gear or the first forward and backward rotating gear rotates, so that an upper part of the first catheter moves in one direction among forward, backward, leftward, and rightward as the direction-adjusting wires are wound or loosened.

5. The device of claim 1, wherein the second catheter is provided with a bending part curved at a predetermined angle at an upper one side thereof in order to more effectively block the orifice of the tricuspid valve.

6. The device of claim 1, wherein the blocking part further comprises:
a supporting wire having both ends thereof coupled to a connecting tube; and
a blocking membrane having one side thereof fixed to the connecting tube and supported by the supporting wire.

7. The device of claim 1, wherein the blocking part further comprises:
a ring-shaped wire installed to enable a connecting tube to pass through and having a central axis thereof provided obliquely with respect to the connecting tube; and
a blocking membrane connecting the connecting tube and the ring-shaped wire to each other.

8. The device of claim 1, wherein the adjusting part comprises a hemostatic valve installed at a lower end of the adjusting part and covering the lower end of the adjusting part, wherein the hemostatic valve prevents an outflow of blood to the lower end of the adjusting part.

9. The device of claim 1, wherein the adjusting part comprises a blood coagulation prevention port provided at a position on one side of the adjusting part and communicating with the adjusting part, wherein the blood coagulation prevention port injects glucose, blood, or physiological saline to prevent the blood inserted into the position-adjustable device for the treatment of the tricuspid regurgitation from coagulating, and to induce the blood to move back into a patient's body.

10. A position-adjustable device for treatment of tricuspid regurgitation, the device comprising:
a first catheter provided with a first lumen;
a second catheter inserted into the first lumen and provided with a second lumen into which a guidewire is inserted;
an adjusting part coupled to lower sides of the first catheter and the second catheter, wherein the adjusting part is provided with a first direction-adjusting device adjusting a direction of an upper end of the first catheter and a second direction-adjusting device adjusting a direction of an upper end of the second catheter; and
a blocking part coupled to an upper side surface of the second catheter and obliquely penetrating and blocking an orifice formed by an incomplete closing of a tricuspid valve.

11. The device of claim 10, wherein, on an inner wall, the first catheter comprises:
a plurality of lumens for first direction-adjusting wires, the lumens provided in parallel to the first lumen and having a closed upper end thereof; and
a first lumen supporter coupled to an upper inner circumferential surface of the first catheter and provided with a protrusion closing upper ends of the plurality of lumens for the first direction-adjusting wires.

12. The device of claim 10, wherein, on the inner wall, the second catheter comprises:
a plurality of lumens for second direction-adjusting wires, the lumens provided in parallel to the second lumen; and
a second lumen supporter coupled to an upper inner circumferential surface of the second catheter and provided with a protrusion closing upper ends of the plurality of lumens for the second direction-adjusting wires.

13. The device of claim 10, wherein the first direction-adjusting device comprises:
a first left and right rotating gear installed therein and rotating left and right;
a pair of first left and right driven gears installed on a left and a right of the first left and right rotating gear so as to perpendicularly engage with the first left and right rotating gear; and
a plurality of first direction-adjusting wires having a lower end thereof coupled to the first left and right driven gear and having an upper end thereof coupled to the first catheter, wherein the first left and right driven gears are rotated when the first left and right rotating gear is rotated left or right, so that an upper part of the first catheter moves to the left or to the right as the direction-adjusting wires are wound or loosened.

14. The device of claim 10, wherein the second direction-adjusting device comprises:
a second left and right rotating gear installed therein and rotating left and right;
a pair of second left and right driven gears installed on a left and a right of the second left and right rotating gear so as to perpendicularly engage with the second left and right rotating gear; and
a plurality of second direction-adjusting wires having a lower end thereof coupled to the second left and right driven gear and having an upper end thereof coupled to the second catheter, wherein the second left and right driven gears are rotated when the second left and right rotating gear is rotated left or right, so that an upper part of the second catheter moves to the left or to the right as the direction-adjusting wires are wound or loosened.

15. The device of claim 10, wherein the first direction-adjusting device comprises:
a first left and right rotating gear installed therein and rotating left and right;
a pair of first left and right driven gears installed on a left and a right of the first left and right rotating gear so as to perpendicularly engage with the first left and right rotating gear;
a first forward and backward rotating gear installed in parallel, spaced apart from the first left and right rotating gear, and rotating left or right;
a pair of first forward and backward driven gears installed so as to perpendicularly engage with the first forward and backward rotating gear at a front and a rear of the first forward and backward rotating gear; and
a plurality of first direction-adjusting wires having a lower end thereof coupled to the first left and right driven gear or the first forward and backward driven gear and having the upper end thereof coupled to the first catheter, wherein the first left and right driven gears or the first forward and backward driven gears are rotated when the first left and right rotating gear or the first forward and backward rotating gear rotates, so that an upper part of the first catheter moves in one direction among forward, backward, leftward, and rightward as the direction-adjusting wires are wound or loosened.

16. The device of claim 10, wherein the second direction-adjusting device comprises:
a second left and right rotating gear installed therein and rotating left and right;
a pair of second left and right driven gears installed at a left and a right of the second left and right rotating gear so as to perpendicularly engage with the second left and right rotating gear;
a second forward and backward rotating gear installed in parallel, spaced apart from the second left and right rotating gear, and rotating left or right;
a pair of second forward and backward driven gears installed so as to perpendicularly engage with the second forward and backward rotating gear at a front and a rear of the second forward and backward rotating gear; and
a plurality of second direction-adjusting wires having a lower end thereof coupled to the second left and right driven gear or the second forward and backward driven gear and having an upper end thereof coupled to the second catheter, wherein first left and right driven gears or first forward and backward driven gears are interlocked and rotated when a first left and right rotating gear or a first forward and backward rotating gear rotates, so that an upper part of the second catheter moves in one direction among forward, backward, leftward, and rightward as the direction-adjusting wires are wound or loosened.

17. The device of claim 10, wherein the blocking part further comprises:
a supporting wire having both ends thereof coupled to a connecting tube; and
a blocking membrane having one side thereof fixed to the connecting tube and supported by the supporting wire.

18. The device of claim 10, wherein the blocking part further comprises:
a ring-shaped wire installed to enable a connecting tube to pass through and having a central axis thereof provided obliquely with respect to the connecting tube; and
a blocking membrane connecting the connecting tube and the ring-shaped wire to each other.

19. The device of claim 10, wherein the adjusting part comprises a hemostatic valve installed at a lower end of the adjusting part and covering the lower end of the adjusting part, wherein the hemostatic valve prevents an outflow of blood to the lower end of the adjusting part.

20. The device of claim 10, wherein the adjusting part comprises a blood coagulation prevention port provided at a position on one side of the adjusting part and communicating with the adjusting part, wherein the blood coagulation prevention port injects glucose, blood, or physiological saline to prevent the blood inserted into the position-adjustable device for the treatment of the tricuspid regurgitation from coagulating, and to induce the blood to move back into patient's body.
